# EUROPEAN PATENT APPLICATION

(11) **EP 4 740 983 A1**
(43) Date of publication of application: **13.05.2026**
(21) Application number: 24836060.4
(22) Date of filing: 03.07.2024
(51) Int. Cl.: A61M 5/303, A61F 9/007, A61M 5/32

(54) **POSITIONAL MISALIGNMENT PREVENTION TOOL AND NEEDLELESS SYRINGE**

(30) Priority: 05.07.2023 JP 2023110824
(71) Applicant: Daicel Corporation, Osaka-shi, Osaka 530-0011 (JP)
(72) Inventor: YAMAMOTO, Yuzo, Tokyo 108-8230 (JP)
(74) Representative: Grünecker Patent- und Rechtsanwälte PartG mbB
(86) International application number: PCT/JP2024/024043
(87) International publication number: WO 2025/009547

(57) **Abstract**

A positional misalignment prevention tool suppresses misalignment of an injector with respect to a target region of an eyeball being an injection target. The injector ejects a substance to be injected from an ejection portion port to the target region. The positional misalignment prevention tool includes a base disposed around an axis along an ejection direction of the substance to be injected, the base surrounding the periphery of the ejection portion, and an irregular portion provided on the target region side of the base.

## Description

### Technical Field

The present invention relates to a positional misalignment prevention tool and a needleless injector.

### Background Art

Patent Document 1 discloses a device that delivers a drug into an eye. The device includes a housing, at least one nozzle provided at a portion of the housing, a supply source of the drug in the housing, and an energy source that provides pressure for discharging the drug to the outside of the housing through the at least one nozzle.

### Citation List

### Patent Document

Patent Document 1: JP 2007-044529 A

### Summary of Invention

### Technical Problem

The device of Patent Document 1 is not equipped with a configuration for suppressing misalignment of an injection position due to movement of a patient or the like, for example, when the drug is injected into the patient. Therefore, there is a problem that a tear may occur when the injection position is misaligned during the injection of the drug.

In view of the above-described circumstances, an object of the present disclosure is to suppress misalignment of an injector with respect to a target region of an eyeball.

### Solution to Problem

In order to solve the problem described above, a positional misalignment prevention tool of the present disclosure is a positional misalignment prevention tool configured to suppress misalignment of an injector with respect to a target region of an eyeball being an injection target. The injector is configured to eject a substance to be injected from an ejection portion to the target region. The positional misalignment prevention tool includes a base disposed around an axis along an ejection direction of the substance to be injected, the base surrounding a periphery of the ejection portion, and an irregular portion provided on the target region side of the base.

In the positional misalignment prevention tool, the injector may be a injector configured to inject the substance to be injected into the target region without using an injection needle, by ejecting the substance to be injected from an ejection port being the ejection portion.

In the positional misalignment prevention tool, the needleless injector may include a housing portion provided with a housing space in which the substance to be injected is housed and the ejection port from which the substance to be injected is ejected to the target region, and the base may be attached to the housing portion of the needleless injector.

In the positional misalignment prevention tool, the needleless injector may include a housing portion provided with a housing space in which the substance to be injected is housed and the ejection port from which the substance to be injected is ejected to the target region, and a holding portion configured to hold the housing portion, and the base may be attached to the holding portion of the needleless injector.

In the positional misalignment prevention tool, the ejection portion of the injector may include an injection needle, and the base may be disposed at a position surrounding the injection needle around the axis.

In the positional misalignment prevention tool, the irregular portion may include a plurality of protrusions around the axis, and a contact surface of each of the protrusions that is to come into contact with the eyeball may be formed along a predetermined curved surface corresponding to the eyeball.

In order to solve the problem described above, a needleless injector of the present disclosure is an injector configured to eject a substance to be injected from an ejection portion into a target region of an eyeball being an injection target. The injector includes a housing portion provided with a housing space in which the substance to be injected is housed and the ejection portion from which the substance to be injected is ejected to the target region, and a positional misalignment prevention tool configured to suppress misalignment of the needleless injector with respect to the target region. The positional misalignment prevention tool includes a base disposed around an axis along an ejection direction of the substance to be injected, the base surrounding a periphery of the ejection portion, and an irregular portion provided on a surface, of the base, facing the target region.

The injector may be a needleless injector configured to inject the substance to be injected into the target region without using an injection needle by ejecting the substance to be injected from an ejection port being the ejection portion.

In the injector, the base may be attached to the housing portion of the needleless injector.

The injector may further include a holding portion configured to hold the housing portion, and the base may be attached to the holding portion of the needleless injector.

In the injector, the ejection portion may include an injection needle, and the base may be disposed at a position surrounding the injection needle around the axis.

In the injector, the irregular portion may include a plurality of protrusions around the axis, and a contact surface of each of the protrusions that is to come into contact with the eyeball may be formed along a predetermined curved surface corresponding to the eyeball.

### Advantageous Effects of Invention

According to the present disclosure, it is possible to suppress misalignment of an injector with respect to a target region of an eyeball.

### Brief Description of Drawings

FIG. 1 is a diagram schematically illustrating the appearance of an injector.
FIG. 2 is a first cross-sectional view of the injector, which is an AA cross section in FIG. 4 described below.
FIG. 3 is a second cross-sectional view of the injector, which is a BB cross section in FIG. 4 described below. The BB cross section is orthogonal to the AA cross section.
FIG. 4 is a diagram illustrating a configuration of a housing that is included in the injector.
FIG. 5 is a diagram illustrating a schematic configuration of an injector assembly.
FIG. 6 is a cross-sectional view illustrating a schematic configuration of a container.
FIG. 7 is an external view illustrating the schematic configuration of the container.
FIG. 8 is a diagram illustrating a schematic configuration of a positional misalignment prevention tool, and is a view (side view) of the positional misalignment prevention tool as viewed from a direction orthogonal to an ejection direction of the injector.
FIG. 9 is a cross-sectional view of FIG. 8.
FIG. 10 is a view of the positional misalignment prevention tool as viewed from a distal end side.
FIG. 11 is a view of the positional misalignment prevention tool as viewed from a base end side.
FIG. 12 is a perspective view illustrating a schematic configuration of the positional misalignment prevention tool.
FIG. 13 is a schematic view illustrating a positional relationship between contact surfaces of the positional misalignment prevention tool and an ejection port.
FIG. 14 is a diagram illustrating a schematic configuration of a positional misalignment prevention tool and a container according to a second embodiment.
FIG. 15 is a diagram for describing a positional relationship between the container and a container holder according to the second embodiment.
FIG. 16 is a cross-sectional view illustrating a schematic configuration of a positional misalignment prevention tool and a container according to a third embodiment.
FIG. 17 is a view of the positional misalignment prevention tool according to the third embodiment as viewed from the distal end side.
FIG. 18 is a schematic configuration diagram of an injector according to a fourth embodiment.
FIG. 19 is a diagram illustrating the distal end side of the positional misalignment prevention tool according to the fourth embodiment.
FIG. 20 is a diagram illustrating the base end side of the positional misalignment prevention tool according to the fourth embodiment.
FIG. 21 is a schematic configuration diagram of an injector according to a fifth embodiment.
FIG. 22 is a diagram illustrating a cross section of the distal end side of an injector.

### Description of Embodiments

### First Embodiment

With reference to the drawings, a needleless injector 1 according to an embodiment of the present disclosure (herein, simply referred to as "injector") is described below. The injector 1 is a needleless injector that ejects an ejection solution, which corresponds to a substance to be injected of the present application, to a target region, using combustion energy of an explosive. In other words, the injector 1 is a device that performs an injection by ejecting the ejection solution to the target region without using an injection needle.

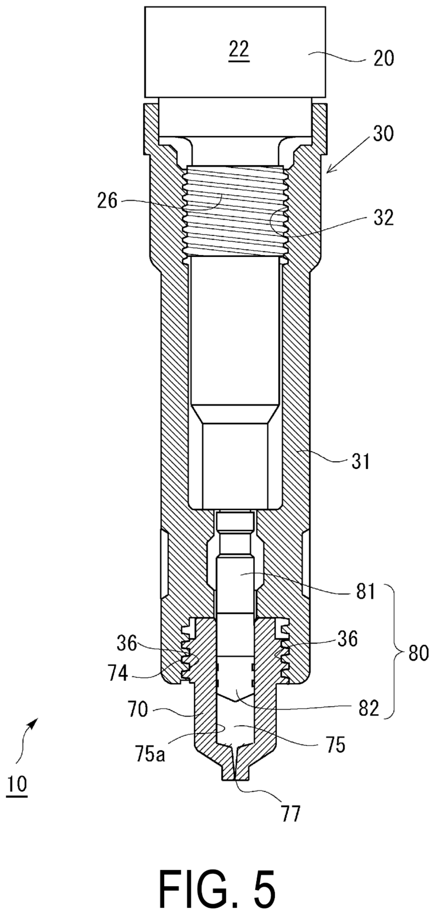

Note that each of the configurations, combinations thereof, and the like in each embodiment are examples, and various additions, omissions, substitutions, and other changes may be made as appropriate without departing from the spirit of the present disclosure. The present disclosure is not limited by the embodiments and is limited only by the claims. Note that, in the present embodiment, as terms indicating a relative positional relationship in a longitudinal direction of the injector 1, "distal end side" and "base end side" are used. The "distal end side" indicates a side closer to the distal end of the injector 1 described later, that is, a position closer to an ejection port 77, and the "base end side" indicates a side in an opposite direction to the "distal end side" in a longitudinal direction of the injector 1, that is, a direction to an igniter 22 side of an injector assembly 10 (see FIG. 5 described later).

### Configuration of Injector 1

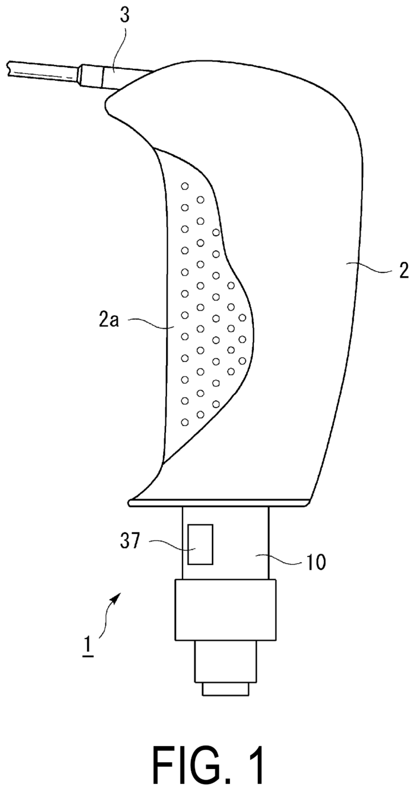

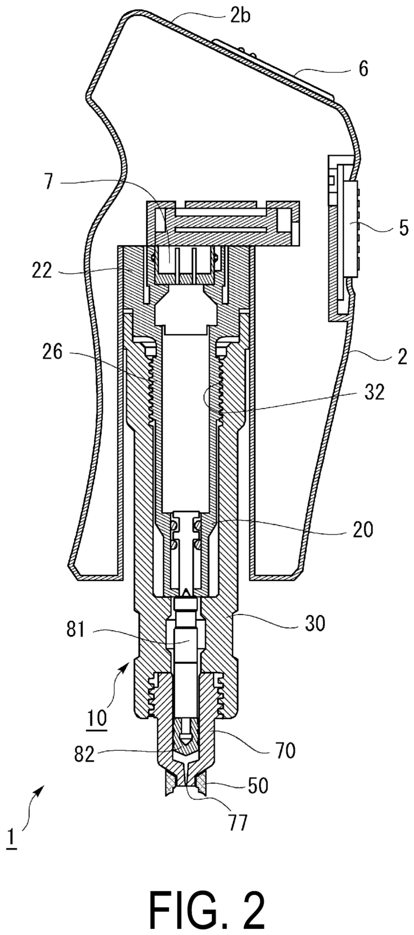

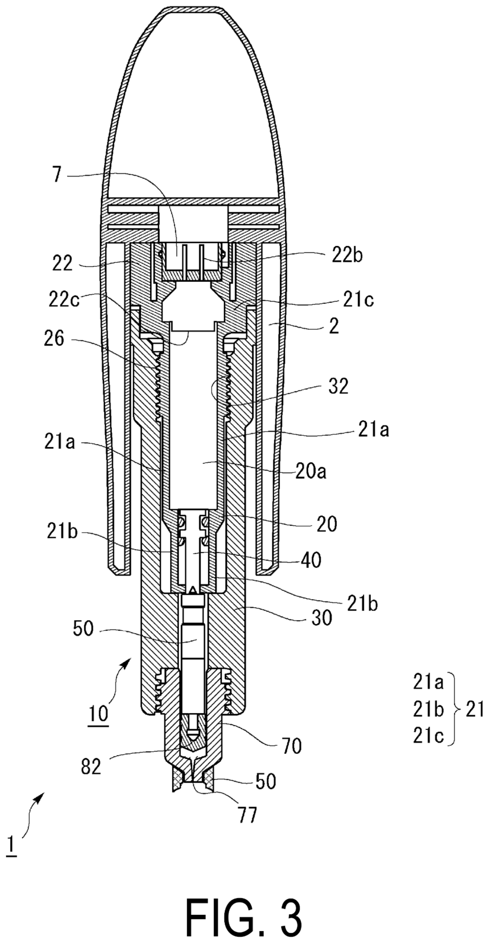

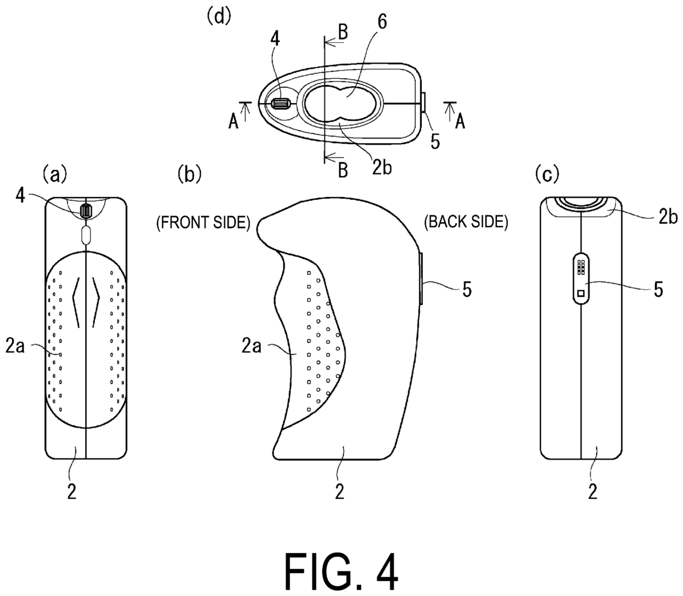

Here, FIG. 1 is a diagram schematically illustrating the appearance of the injector 1. FIG. 2 is a first cross-sectional view of the injector 1, which is an AA cross section in FIG. 4 described later. FIG. 3 is a second cross-sectional view of the injector 1, which is a BB cross section in FIG. 4 described later. The BB cross section is orthogonal to the AA cross section. FIG. 4 is a diagram illustrating a configuration of the housing 2 that is included in the injector 1, and FIG. 5 is a diagram illustrating a schematic configuration of the injector assembly 10. The injector 1 is formed by the injector assembly 10 being attached to the housing 2. A power cable 3 (FIG. 1) for supplying a drive current to the igniter 22 in the injector assembly 10 is connected to the housing 2.

Note that, in the following description in the present application, the ejection solution ejected to the target region by the injector 1 is formed of a liquid medium including a predetermined substance, which exerts an effect or a function expected in the target region. In the ejection solution, the predetermined substance may be in a state of being dissolved in liquid being a medium, or may be in a state of being simply mixed instead of being dissolved.

For example, examples of the predetermined substance included in the ejection solution include an organism-derived substance and a substance with a desired bioactivity, which can be ejected to the target region being an organism. For example, examples of the organism-derived substance include DNA, RNA, a nucleic acid, an antibody, and a cell. Examples of the substance with a desired bioactivity include various substances exerting pharmacological or therapeutic effects, which are exemplified by medicines composed of low molecular compounds, proteins, peptides, or the like, a vaccine, an inorganic substance such as metal particles for thermotherapy or radiotherapy, and a carrying body functioning as a carrier. Further, the liquid being the medium of the ejection solution is only required to be a substance suitable for administering the predetermined substance exemplified by those substances to the target region, and may be aqueous or oleaginous, which is not limited. Further, viscosity of the liquid being the medium is not particularly limited as long as the predetermined substance can be ejected by the injector 1.

In the injector 1, the injector assembly 10 is configured to be attachable to and detachable from the housing 2 freely. A housing space 75 (see FIG. 5) formed between a container (housing portion) 70 and a plunger 80 in the injector assembly 10 is filled with the ejection solution during a preparation stage, which is a stage before the operation of the injector 1. The injector assembly 10 is a unit that is replaced each time the ejection solution is ejected. The injector assembly 10 will be described in detail below.

On the other hand, the housing 2 has a grip portion 2a formed to be gripped by a user of the injector 1 in use, and is provided with a plurality of switches for operating the injector 1 to eject the ejection solution. Note that the injector 1 is configured to be capable of being held and operated by one hand of the user. In this context, the housing 2 will be described with reference to FIG. 4. In FIG. 4, (a) illustrates the outer appearance of the housing 2 as viewed from the front side, (b) illustrates the outer appearance of the housing 2 as viewed from one side, (c) illustrates the outer appearance of the housing 2 as viewed from the back side, and (d) illustrates the outer appearance of the housing 2 as viewed from the upper side. Here, "front side" indicates a portion positioned on the distal side of the user holding the housing 2, which is the left side in FIG. 4(b), and "back side" indicates a portion positioned on the proximal side of the user holding the housing 2, which is the right side in FIG. 4(b). Thus, when the user holds the housing 2 with one hand, fingertips rest on the front side of the distal housing 2 which is the distal side, and the wrist is in the vicinity of the back side of the housing 2 which is the proximal side. The "upper side" is a portion of the injector 1 on the base end side.

Considering such a way of holding by the user, the grip portion 2a is provided at a front side portion of the housing 2 so that the user can easily rest his or her fingertips thereon. The grip portion 2a is provided with a plurality of dimples making the user's fingertips even easier to be rested thereon. Furthermore, the grip portion 2a has gentle recesses and protrusions on the front side of its outer shell (see (b) in FIG. 4) so that the user's forefinger and middle finger can be easily rested thereon, for the sake of more stable holding of the housing by the user.

Further, the housing 2 is provided with a first switch 5 and a second switch 6 that are two operating switches for operating the injector 1. A first switch 5 and a second switch 6 are connected to a control unit, such as a microcomputer (not illustrated). The control unit controls the supply of ignition current to the igniter 22 based on a signal from each switch, thereby controlling an operation of the injector 1. The first switch 5 is a sliding switch provided on the back side of the housing 2, the sliding direction of which being an upward and downward direction of the housing 2 (direction between the distal end and the base end). The first switch 5 is constantly biased in the upward direction. The user can achieve a standby state of the injector 1 by continuously sliding the first switch 5 downward (toward the distal end side) for a predetermined period of time against the biasing force. The standby state is a state in which the injector 1 is ready to eject the ejection solution. When a user makes an additional operation in this state, the ejection is implemented.

The second switch 6 is a press type switch provided on an inclined surface 2b on the upper side of the housing 2. The user can press the second switch 6 in a direction toward the inner side of the housing 2. The control unit is configured to supply an ignition current to the igniter 22 in response to the pressing operation on the second switch 6 while the injector 1 is in the standby state as a result of the operation on the first switch 5 described above. A connector 4 to which the power cable 3 is connected is provided on the front side of the inclined surface 2b on the upper side of the housing 2. In the present embodiment, the connector 4 is a USB connector, and the power cable 3 is freely attachable to and detachable from the housing 2.

Note that, as described above, in the present embodiment, the power for actuating the igniter 22 is supplied from the outside through the power cable 3. Alternatively, a battery for supplying such power may be provided inside the housing 2. In this case, the housing 2 can be repeatedly used while replacing the injector assembly 10, until the battery runs out. When the battery runs out, the battery may be replaced.

A schematic configuration of the injector assembly 10 is illustrated in FIG. 5. The injector assembly 10 is attached to the housing 2 to form the injector 1, as illustrated in FIGS. 2 and 3. Specifically, the injector assembly 10 is an assembly including an actuator (drive unit) 20, an attachment 30, a positional misalignment prevention tool 50, the container 70, and the plunger 80. How the injector assembly 10 is assembled will be described below.

The actuator 20 has a body 21 formed in a cylindrical shape. The body 21 includes a center portion 21a in the center thereof, a distal end portion 21b on the distal end side thereof, and a base end portion 21c on the base end side thereof. The distal end portion 21b, the center portion 21a, and the base end portion 21c of the body 21 have their internal spaces in communication with each other. The distal end portion 21b has an opening 27 on the distal end side. Here, the igniter 22, which is an electric igniter that generates ejection energy through combustion of an ignition agent, is attached to the base end portion 21c of the body 21. The igniter 22 has an ignition pin 22b to which ignition current is supplied from the outside. The ignition pin 22b is coupled to a socket 7 on the side of the housing 2 in a state in which the injector assembly 10 is attached to the housing 2. The attachment state of the igniter 22 to the body 21 is determined such that a combustion product generated by the operation of the igniter 22 is discharged toward the center portion 21a of the body 21. Specifically, the igniter 22 is attached to the base end portion 21c of the body 21 to have a discharge surface 22c, from which the combustion product is discharged, directed toward the center portion 21a.

Herein, a combustion energy used in the igniter 22 for the ignition charge is an energy for the injector 1 to eject the ejection solution to the target region. Note that, examples of the ignition charge include an explosive containing zirconium and potassium perchlorate (ZPP), an explosive containing titanium hydride and potassium perchlorate (THPP), an explosive containing titanium and potassium perchlorate (TiPP), an explosive containing aluminum and potassium perchlorate (APP), an explosive containing aluminum and bismuth oxide (ABO), an explosive containing aluminum and molybdenum oxide (AMO), an explosive containing aluminum and copper oxide (ACO), an explosive containing aluminum and iron oxide (AFO), and an explosive composed of a combination of a plurality of these explosives. These explosives exhibit characteristics that, although the explosives generate high-temperature and high-pressure plasma during combustion immediately after ignition, when the combustion product condenses at room temperature, the explosives do not contain gaseous components and hence the pressure generated decreases abruptly. Note that an explosive other than these may be used as the ignition agent as long as appropriate ejection of the ejection solution can be performed.

The body 21 is a cylindrical member, and the internal space of the center portion 21a is a combustion chamber 20a. Furthermore, a male thread portion 26 is formed in a part of the outer surface of the center portion 21a. The male thread portion 26 is configured to mate with a female thread portion 32 of the attachment 30 described below. The effective lengths of the male thread portion 26 and the female thread portion 32 are determined to guarantee sufficient coupling force therebetween. The internal space of the distal end portion 21b adjacent to the center portion 21a is formed in a cylindrical shape in which a piston 40 is slidably disposed.

Then, when the igniter 22 is actuated, the combustion product is discharged into the combustion chamber 20a, the pressure therein rises, and the piston 40 receives the pressure and results in sliding toward the distal end side. Thus, the actuator 20 has a mechanism with the igniter 22 serving as an actuation source and the piston 40 serving as an output unit.

The attachment 30 is a member for attaching the actuator 20, the plunger 80, and the container 70 as illustrated in FIG. 5. For a body 31 of the attachment 30, a resin such as nylon 6-12, polyarylate, polybutylene terephthalate, polyphenylene sulfide, or a liquid crystal polymer, which are known, may be used, for example. Further, filler such as glass fibers and glass filler may be contained in those resins. 20 to 80 mass% of glass fibers may be contained in polybutylene terephthalate, 20 to 80 mass% of glass fibers may be contained in polyphenylene sulfide, or 20 to 80 mass% of minerals may be contained in a liquid crystal polymer. Note that the material of the attachment 30 is not limited to a resin, and may be a metal or a ceramic.

Of the internal space of the body 31, in a region extending from the base end side to the center, the actuator 20 is disposed as illustrated in FIG. 5. Then, of the internal space where the actuator 20 is disposed, in a region on the base end side, the base end portion 21c of the actuator 20 is generally positioned, and in a region on the distal end side whose diameter is smaller than that of the base end side, the center portion 21a and the distal end portion 21b of the actuator 20 are generally positioned. Further, the female thread portion 32 is disposed at the inner wall surface of the body 31, and the female thread portion 32 is formed in a manner that it engages with the male thread portion 26 provided at the center portion 21a of the actuator 20.

Further, of the internal space of the body 31, in a region on the distal end side with respect to the region where the actuator 20 is disposed, the plunger 80 is generally disposed as illustrated in FIG. 5. The diameter of the region where the plunger 80 is disposed is smaller than the diameter of the region where the distal end portion 21b of the actuator 20 is disposed, and is a diameter that allows the plunger 80 to slide.

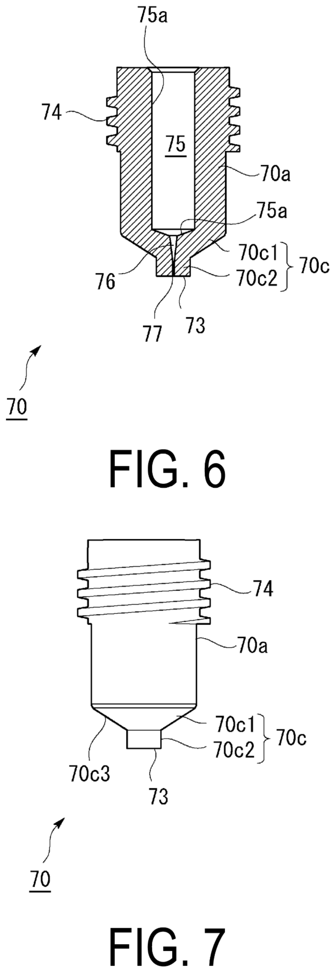

Further, of the internal space of the body 31, in a region on the distal end side, a part of the container 70 is generally disposed. The region where the container 70 is disposed communicates with the region where the plunger 80 is disposed on the base end side of the region, and the distal end side of the region where the container 70 is disposed is open to the distal end surface of the attachment 30. A female thread portion 36 for attaching the container 70 is formed at the inner wall of the region where the container 70 is disposed. The female thread portion 36 is screwed with a male thread portion 74 of the container 70 illustrated in FIGS. 6 and 7 described below, whereby the attachment 30 and the container 70 are coupled to each other.

Next, the plunger 80 will be described. The plunger 80 is a member that pressurizes the ejection solution using an energy received from the piston 40, and includes a plunger rod 81 and a stopper portion 82. The plunger rod 81 is formed using, for example, a resin material suitable for pressurizing the plunger rod 81, but is not limited thereto, and may be formed using, for example, the same type of material as that of the attachment 30.

The stopper portion 82 formed using an elastic member such as rubber is attached to the distal end side of the plunger rod 81. Note that specific examples of the material of the stopper portion 82 include butyl rubber and silicon rubber. Further, there may be exemplified a styrene-based elastomer or a hydrogenated styrene-based elastomer, or a substance obtained by mixing a styrene-based elastomer or a hydrogenated styrene-based elastomer with polyolefin such as polyethylene, polypropylene, polybutene, and an α-olefin copolymer, oil such as liquid paraffin and process oil, or a powder inorganic substance such as talc, cast, and mica. Further, as the material of the stopper portion 82, a polyvinyl chloride-based elastomer, an olefin-based elastomer, a polyester-based elastomer, a polyamide-based elastomer, a polyurethane-based elastomer, any of various rubber materials (particularly, a vulcanized material) such as natural rubber, isoprene rubber, chloroprene rubber, nitrile butadiene rubber, and styrene butadiene rubber, or a mixture thereof may be employed. Furthermore, the stopper portion 82 pressurizes the ejection solution by sliding inside the container 70 described below. Thus, a surface of the stopper portion 82 and an inner wall surface 75a of the container 70 may be coated or processed using any of various substances, to secure and adjust slidability between the stopper portion 82 and the inner wall surface 75a of the housing space 75 of the container 70. Examples of the coating agent may include polytetrafluoroethylene (PTFE), silicone oil, diamond-like carbon, nanodiamond, and the like.

FIG. 6 is a cross-sectional view illustrating a schematic configuration of the container 70, and FIG. 7 is an external view illustrating the schematic configuration of the container 70. The container 70 is a member that houses the ejection solution and defines a flow path for ejecting, to the target region, the ejection solution pressurized by the plunger 80. The container 70 is formed using a material selected in consideration of the pressurization by the plunger and the definition of the flow path. The container 70 of the present embodiment is formed using a resin material. Note that the container 70 is not limited to being formed using the resin material, and may be formed using, for example, the same type of material as that of the attachment 30.

The container 70 includes the housing space 75 that can house the ejection solution and is formed in a manner that the stopper portion 82 of the plunger 80 can move therein, and a flow path 76 that connects the housing space 75 to the ejection port (ejection portion) 77 facing the outside of the container 70. Specifically, the container 70 includes a body portion 70a having a cylindrical shape defining the housing space 75, and a nozzle portion 70c that is connected to the distal end side of the body portion 70a and defines the flow path 76. Further, the nozzle portion 70c includes a distal end portion 70c2 internally including a part of the flow path 76 on the ejection port 77 side, and a tapered portion 70c1 that connects the distal end portion 70c2 and the body portion 70a to each other and includes a distal end side outer surface 70c3 inclined with respect to the center axis in the longitudinal direction of the attachment 30. Then, the positional misalignment prevention tool 50 described below is externally fitted to the outer periphery of the distal end portion 70c2.

In the injector assembly 10, as illustrated in FIG. 5, the distal end side of the plunger 80 is fitted into the housing space 75 of the container 70 in a manner that the stopper portion 82 of the plunger 80 can slide inside the housing space 75 in a direction toward the nozzle portion 70c (direction toward the distal end side). In a state where the plunger 80 is fitted into the container 70, a space formed between the stopper portion 82 of the plunger 80 and the container 70 becomes a storage space of the ejection solution, that is, a space in which the ejection solution is encapsulated. In other words, when the plunger 80 is fitted into the initial position of the housing space 75 in the container 70, the distal end surface of the stopper portion 82 and the inner wall surface, of the container 70, located further to the distal end side than the distal end surface of the stopper portion 82 define the storage space. The flow path of the container 70 opens in a distal end surface 73 of the nozzle portion 70c to form the ejection port 77. Therefore, when the plunger 80 slides inside the housing space 75, the ejection solution housed in the housing space 75 is pressurized and the ejection solution is ejected from the ejection port 77 through the flow path 76.

The flow path 76 provided in the container 70 has an inner diameter smaller than that of the housing space 75. With this configuration, the ejection solution that has been applied with a high pressure is ejected to the outside through the ejection port 77. Further, the male thread portion 74 for attaching the container 70 to the attachment 30 is formed at the outer periphery on the base end side of the container 70. The male thread portion 74 is screwed with the female thread portion 36 of the attachment 30.

The distal end shape of the stopper portion 82 of the plunger 80 is formed generally matching the distal end shape of the housing space 75, which is defined by the inner wall surface 75a near a portion at which the housing space 75 and the flow path 76 are connected to each other (the deepest part of the housing space 75). In the present embodiment, both the distal end shape of the stopper portion 82 and the distal end shape of the housing space 75 are tapered shapes that decrease in diameter toward the distal end side. With this configuration, a smallest possible gap can be formed between the stopper portion 82 and the inner wall surface 75a of the container 70 when the plunger 80 slides for ejecting the ejection solution and reaches the deepest part of the housing space 75, whereby the ejection solution can be prevented from wastefully remaining in the housing space 75. However, the shape of the stopper portion 82 is not limited to a particular shape as long as desired effects can be obtained with the injector 1 of the present embodiment. Further, the stopper portion 82 is formed having an outer diameter slightly larger than that of the housing space 75 of the container 70, and thus, when the stopper portion 82 is fitted into the housing space 75 in a state of being compressed in the radial direction, the stopper portion 82 suitably comes into contact with the inner wall surface 75a in a manner that the airtightness is maintained between the stopper portion 82 and the container 70. Note that the stopper portion 82 need not necessarily be formed having a larger outer diameter than the housing space 75 and, for example, may have substantially the same diameter as the housing space 75, as long as the ejection solution can be appropriately ejected.

### Assembly of Injector 1

With regard to the assembly of the injector 1, first, how the injector assembly 10 is assembled will be described. In a state where the stopper portion 82 of the plunger 80 is inserted to the deepest part of the housing space 75 of the container 70, the plunger 80 is retracted with the ejection port 77 of the container 70 in communication with the ejection solution. Since the stopper portion 82 and the inner wall surface 75a of the housing space 75 are in close contact with each other, the retraction action produces a negative pressure in the housing space. Thus, the housing space 75 can be filled with the ejection solution through the ejection port 77. At this time, the plunger 80 is retracted to an extent enough for making the part of the plunger 80 protruding from the container 70 (plunger rod 81) reach the region where the piston 40 is disposed in the internal space of the attachment 30, when the container 70 is attached to the attachment 30 in this state.

When the container 70 with the housing space 75 filled with the ejection solution is attached to the attachment 30, the actuator 20 is further inserted into the attachment 30 from the base end side. The actuator 20 is inserted until the distal end surface of the piston 40 disposed at the distal end portion 21b of the actuator 20 reaches the base end surface of the plunger 80 inside the attachment 30. At this time, the male thread portion 26 provided at the center portion 21a of the actuator 20 is screwed with the female thread portion 32 of the attachment 30, whereby the actuator 20 and the attachment 30 are suitably coupled to each other. At this time, the piston 40 incorporated in the actuator 20 is connected to the plunger 80. Note that the connection between the piston 40 and the plunger 80 is not limited to simple abutment, and fitting portions may be provided at the distal end of the piston 40 and the base end of the plunger 80 and fitted to each other.

When the actuator 20 is attached to the attachment 30 to which the container 70 and the plunger 80 are attached as described above, the plunger 80 is pushed in a manner that it moves from the piston 40 toward the distal end side, whereby the plunger 80 is positioned at a predetermined position (pre-operation position) inside the container 70. At this time, in accordance with the pushing of the plunger 80, the excess ejection solution in the container 70 is discharged from the ejection port 77, and a predetermined amount of the ejection solution remains in the container 70. Note that the volume of the space for housing the ejection solution formed between the plunger 80 positioned at the pre-operation position and the container 70 is determined and set to a volume that secures an appropriate ejection amount of the ejection solution when the injector 1 is operated. Therefore, when the injector assembly 10 is assembled as described above, an amount of the ejection solution housed in the housing space 75 of the container 70, that is, an amount of the ejection solution to be ejected is set to the predetermined amount determined in advance.

The injector assembly 10 having the above-described configuration can be loaded into the housing 2 with the ignition pin 22b of the igniter 22 fitted into the socket 7 on the housing 2 side. As a result, the usable injector 1 is prepared (see FIGS. 1 to 3). The user holds the housing 2 of such injector 1 with one hand and slides the first switch 5 located on the back side of the housing 2 for a predetermined period of time, putting the injector 1 in the standby state. In this state, when the user presses the second switch 6 with the ejection port 77 being in contact with the target region, the igniter 22 is actuated, and the ejection solution is pressurized via the piston 40 and the plunger 80. Thus, the ejection is implemented, and the ejection solution is injected into the target region.

### Configuration of Positional Misalignment Prevention Tool

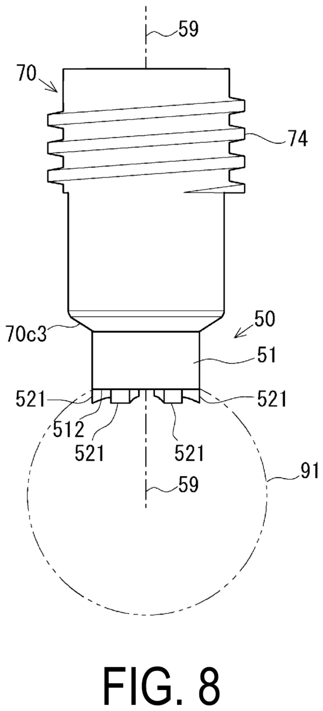

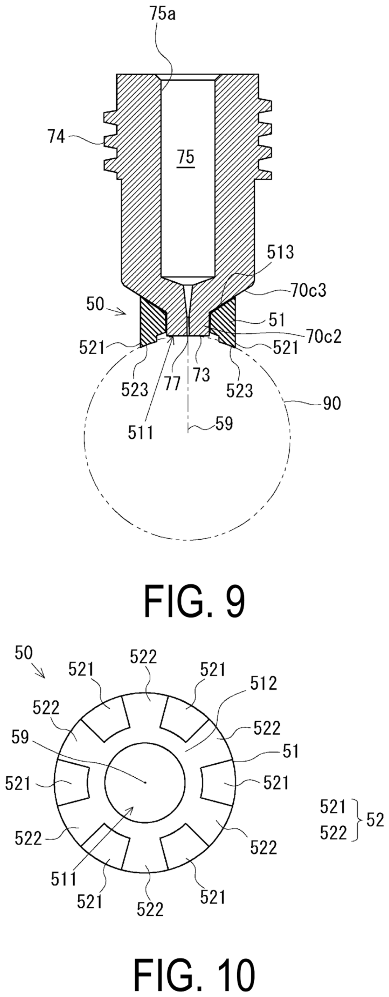

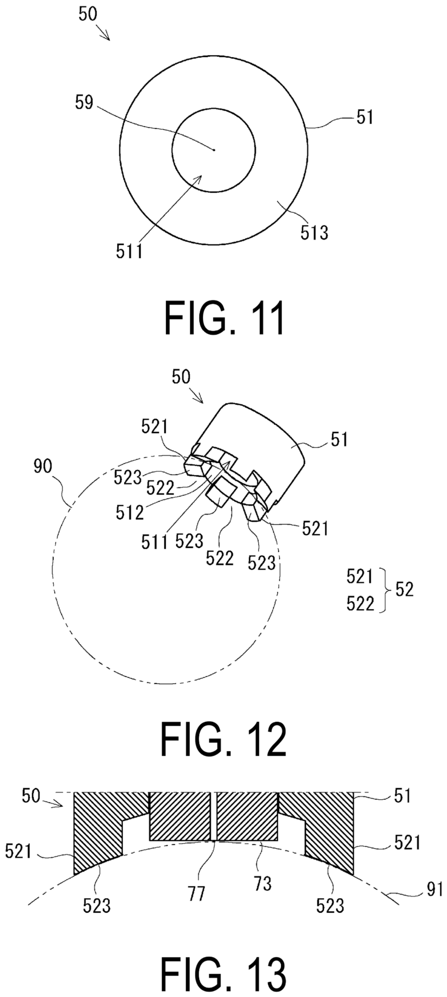

Next, the positional misalignment prevention tool 50 provided at the distal end of the injector 1 will be described. FIG. 8 is a diagram illustrating a schematic configuration of the positional misalignment prevention tool 50, and is a view (side view) of the positional misalignment prevention tool 50 as viewed from a direction orthogonal to an ejection direction of the injector 1. FIG. 9 is a cross-sectional view of FIG. 8, FIG. 10 is a view of the positional misalignment prevention tool 50 as viewed from the distal end side, FIG. 11 is a view of the positional misalignment prevention tool 50 as viewed from the base end side, and FIG. 12 is a perspective view illustrating the schematic configuration of the positional misalignment prevention tool 50.

The positional misalignment prevention tool 50 includes a base 51 disposed around an axis 59 along the ejection direction of the ejection solution in a manner that the base 51 surrounds the periphery of the ejection port 77, and an irregular portion 52 provided on the target region side of the base 51.

The base 51 is an annular member, and is externally fitted to the distal end portion 70c2 of the nozzle portion 70c by the distal end portion 70c2 passing through an inner space 511. That is, the positional misalignment prevention tool 50 of the present embodiment is attached to the container 70 of the injector 1. The base 51 of the present embodiment has a circular shape, but is not limited thereto, and may have a shape in which a part of a circle is missing, such as a C shape. Note that the positional misalignment prevention tool 50 may be coupled by another coupling mechanism such as a screw or snap-fit, instead of fitting. Further, the positional misalignment prevention tool 50 of the present embodiment is detachably attached, but is not limited thereto, and may be fixedly attached to the container 70 by adhesion, welding, or the like. Further, the attachment of the positional misalignment prevention tool 50 is not limited to the joining of the positional misalignment prevention tool 50 and the container 70 that are separately formed, and the positional misalignment prevention tool 50 and the container 70 may be formed integrally.

A surface (base end surface) 513 on the base end side of the base 51 is formed in a tapered shape in a manner that it follows the distal end side outer surface (tapered surface) 70c3 of the tapered portion 70c1 of the nozzle portion 70c.

In the irregular portion 52, a plurality of protruding portions 521 are erected on a surface (base surface) 512, of the base 51, facing the target region. The plurality of protruding portions 521 are provided at intervals in the circumferential direction of the base surface 512. Accordingly, a recessed portion 522 is formed between the protruding portions 521. Note that, in the present embodiment, the intervals between the corresponding protruding portions 521 are equal, and the protruding portions 521 are arranged in rotational symmetry about the axis 59.

In FIGS. 8, 9, and 12, the reference sign 90 indicates a region where an eyeball, which is an injection target, is located when the injector 1 is in use. With the positional misalignment prevention tool 50, when the ejection port 77 is pressed against the eyeball during use of the injector 1, a contact surface 523 on the distal end side of the protruding portion 521 comes into contact with the eyeball. Therefore, the contact surface 523 of each of the protruding portions 521 is formed along a predetermined curved surface corresponding to the eyeball. Here, the predetermined curved surface is, for example, a spherical surface. By forming the contact surface 523 along a spherical surface of an eyeball having an average diameter, the positional misalignment prevention tool 50 can be used for general purposes. Further, the contact surface 523 may be formed along a spherical surface of an eyeball having a diameter slightly larger than the average eyeball diameter. In this case, when the positional misalignment prevention tool 50 is pressed against the eyeball, the inner side of the contact surface 523 may mainly come into contact with the eyeball in a case where the eyeball has a diameter equal to or smaller than the average eyeball diameter, and the outer side of the contact surface 523 may mainly come into contact with the eyeball in a case where the eyeball has a diameter larger than the average eyeball diameter. Note that the predetermined curved surface along which the contact surface 523 is formed is not limited to the spherical surface, and may be a curved surface that deviates from the spherical surface, as long as the contact surface 523 of each of the protruding portions 521 comes into contact with the eyeball. For example, the contact surface 523 may be formed along an aspherical surface having a high curvature on the axis 59 side (inner side) and a low curvature on the side away from the axis 59 (outer side) in the radial direction of the positional misalignment prevention tool 50.

FIG. 13 is a schematic view illustrating a positional relationship between the contact surfaces 523 of the positional misalignment prevention tool 50 and the ejection port 77. As illustrated in FIG. 13, the contact surfaces 523 of the positional misalignment prevention tool 50 and the ejection port 77 are disposed along a predetermined curved surface 91. Therefore, when the ejection port 77 of the injector 1 is pressed against the eyeball, the contact surface 523 of each of the protruding portions 521 comes into contact with the eyeball. Note that the contact surfaces 523 and the ejection port 77 are not strictly limited to the configuration in which they are disposed on the predetermined curved surface 91. For example, the ejection port 77 may be disposed at a position separated from the predetermined curved surface 91 toward the base end side by a predetermined distance in consideration of a distance (predetermined distance) by which the protruding portion 521 bites into the surface of the eyeball. Accordingly, when the ejection port 77 of the injector 1 is pressed against the eyeball, the ejection port 77 can come into contact with the surface of the eyeball in a state where each of the protruding portions 521 is bitten into the eyeball.

As described above, according to the present embodiment, when the ejection port 77 is pressed against the eyeball during use of the injector 1, the irregular portion 52 of the positional misalignment prevention tool 50 comes into contact with the surface of the eyeball to increase the frictional force as compared with a case where the ejection port 77 is simply pressed against the eyeball, and thus the misalignment of the injector 1 with respect to the target region of the eyeball can be suppressed.

Further, with the positional misalignment prevention tool 50, since the protruding portion 521 comes into contact with the eyeball at a position away from the ejection port 77 in the radial direction, the contact between the surface of the eyeball and the distal end of the injector is stabilized, and the misalignment is suppressed.

Furthermore, with the positional misalignment prevention tool 50, since the protruding portions 521 of the irregular portion 52 are arranged in rotational symmetry, the force generated when the injector 1 is pressed against the eyeball is evenly applied to the periphery of the ejection port 77, and thus the misalignment of the injector 1 is effectively suppressed.

### Second Embodiment

The present embodiment is different from the first embodiment in that a container holder 71 that holds the container 70 is provided and a positional misalignment prevention tool 50A is attached to the container holder (holding portion) 71. Note that since other portions are the same as those of the first embodiment described above, the same elements are denoted by the same reference signs and description thereof will not be repeated.

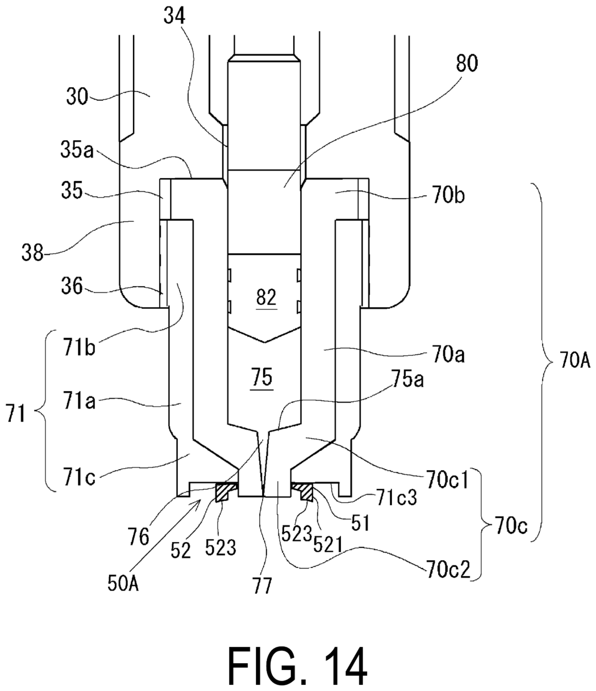

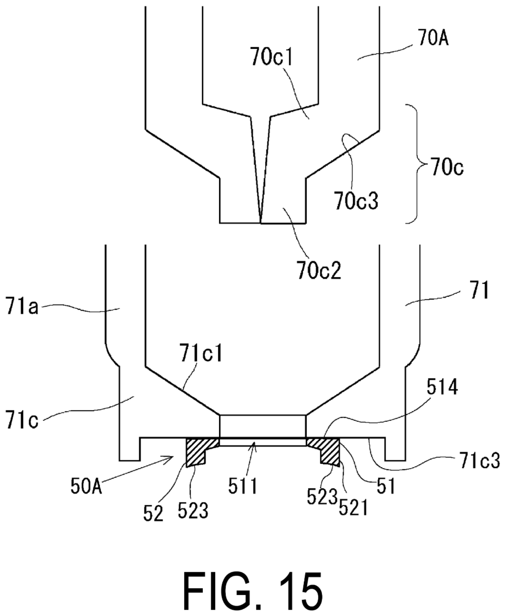

FIG. 14 is a diagram illustrating a schematic configuration of the positional misalignment prevention tool 50A and the container 70A according to the second embodiment. FIG. 15 is a diagram for describing a positional relationship between the container 70A and the container holder 71 according to the second embodiment.

The container 70A itself of the present embodiment does not include a male thread portion that mates with the female thread portion 36 formed at the inner wall on the distal end side of the attachment 30. Thus, the container 70A is attached to the attachment 30 by the container holder 71. The container holder 71 includes a body portion 71a that defines a space containing the container 70A, a mating portion 71b that is formed with a male thread that mates with the female thread portion 36 connected to the base end side of the body portion 71a, and a contact portion 71c that is connected to the distal end side of the body portion 71a and includes an inner surface 71c1 (see FIG. 15) that comes into surface contact with the distal end side outer surface 70c3 of the container 70A. Further, as illustrated in FIG. 15, the contact portion 71c is provided with an opening 71c2 through which the distal end portion 70c2 of the container 70A can pass.

The positional misalignment prevention tool 50A is provided at the distal end surface 71c3 of the container holder 71. The positional misalignment prevention tool 50A includes the base 51 disposed around the axis 59 along the ejection direction of the ejection solution in a manner that the base 51 surrounds the periphery of the opening 71c2, and the irregular portion 52 provided on the target region side of the base 51. A surface (base end surface) 514 on the base end side of the base 51 is attached to the distal end surface 71c3 of the container holder 71 by adhesion, welding, or the like. Note that, although the positional misalignment prevention tool 50 of the present embodiment is fixedly attached to the container holder 71, the positional misalignment prevention tool 50 may be detachably attached to the container holder 71 via a screw or a fitting portion. Further, the attachment of the positional misalignment prevention tool 50 is not limited to the joining of the positional misalignment prevention tool 50 and the container holder 71 that are separately formed, and the positional misalignment prevention tool 50 and the container holder 71 may be formed integrally.

The container 70A is housed with the distal end portion 70c2 passing through the opening 71c2 of the container holder 71 having the above-described configuration. At this time, the container 70A is in a state where the housing space 75 is filled with the substance to be injected and the plunger 80 is loaded. In this housed state, the distal end side outer surface 70c3 of the container 70A and the inner surface 71c1 of the container holder 71 are in surface contact with each other on the distal end side, and the base end portion 70b of the container 70A and the mating portion 71b of the container holder 71 are in contact with each other on the base end side. Then, while the container 70A is housed in the container holder 71, the male thread formed at the mating portion 71b of the container holder 71 and the female thread portion 36 of the attachment 30 are mated until the base end side end surface of the base end portion 70b of the container 70A comes into contact with an end surface 35a of a third region 35 of the attachment 30. As a result, with the fastening force produced by the mating, the container 70A is attached to the attachment 30 with the distal end side outer surface 70c3 of the container 70A pressed by the container holder 71 from the distal end side toward the base end side, that is, in a direction opposite to a movement direction of the plunger 80. At this time, the plunger 80 is in a state of being disposed inside both the housing space 75 in the container 70A and the region where the plunger 80 is generally disposed in the attachment 30.

The steps of assembling the injector assembly 10 by attaching the actuator 20 to the attachment 30 in this state, and the like, and the steps of assembling the injector 1 are the same as those of the first embodiment described above.

As described above, the injector 1 of the present embodiment includes the positional misalignment prevention tool 50A at the distal end of the container holder 71, and when the ejection port 77 is pressed against the eyeball during use of the injector 1, the irregular portion 52 of the positional misalignment prevention tool 50A comes into contact with the surface of the eyeball to suppress the misalignment of the injector 1.

### Third Embodiment

The present embodiment is different from the first embodiment described above in that the configuration of an irregular portion 52B is different. Note that since other portions are the same as those of the first embodiment described above, the same elements are denoted by the same reference signs and description thereof will not be repeated.

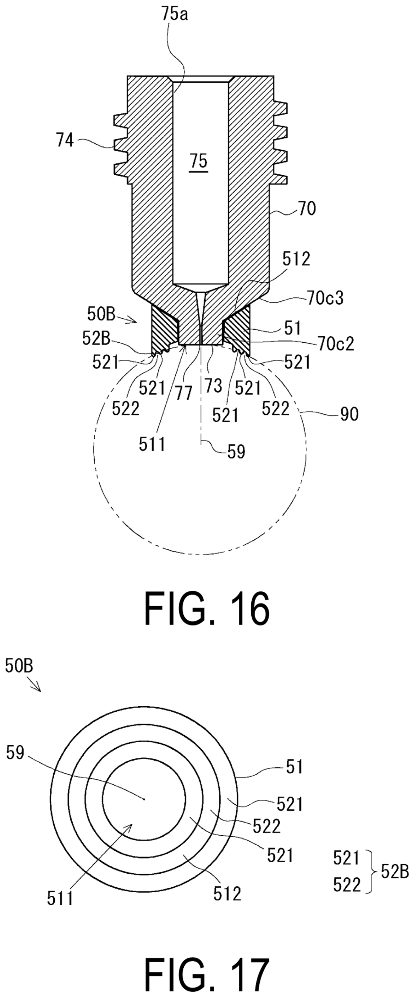

FIG. 16 is a cross-sectional view illustrating a schematic configuration of a positional misalignment prevention tool 50B and the container 70 according to the third embodiment, and FIG. 17 is a view of the positional misalignment prevention tool 50B as viewed from the distal end side. As illustrated in FIGS. 16 and 17, the positional misalignment prevention tool 50B of the present embodiment includes the base 51 surrounding the periphery of the ejection port 77, and the irregular portion 52B provided on the target region side of the base 51.

The irregular portion 52B is formed by concentrically erecting a plurality of protruding portions 521B on the surface (base surface) 512 facing the target region of the base 51. The protruding portions 521B are provided at intervals in the radial direction of the base surface 512. As a result, a recessed portion 522B is formed between the protruding portions 521B. Note that, in the present embodiment, the intervals between the corresponding protruding portions 521B are equal, and the protruding portions 521B are arranged in rotational symmetry about the axis 59. The protruding portions 521B are formed in a circular shape when viewed from the distal end side, but may be provided being divided into a plurality of portions in a manner that they have a shape in which a part of a circle is missing.

As described above, according to the present embodiment, when the ejection port 77 is pressed against the eyeball during use of the injector 1, the irregular portion 52B of the positional misalignment prevention tool 50B comes into contact with the surface of the eyeball and increases the frictional force as compared with the case where the ejection port 77 is simply pressed against the eyeball, and thus the misalignment of the injector 1 with respect to the target region of the eyeball can be suppressed.

### Fourth Embodiment

The present embodiment is different from the first embodiment in that an injector 1C is not a needleless injector but an injector with a needle, and a positional misalignment prevention tool 50C is attached to a needle base of the injector. Note that since other portions are the same as those of the first embodiment described above, the same elements are denoted by the same reference signs and description thereof will not be repeated.

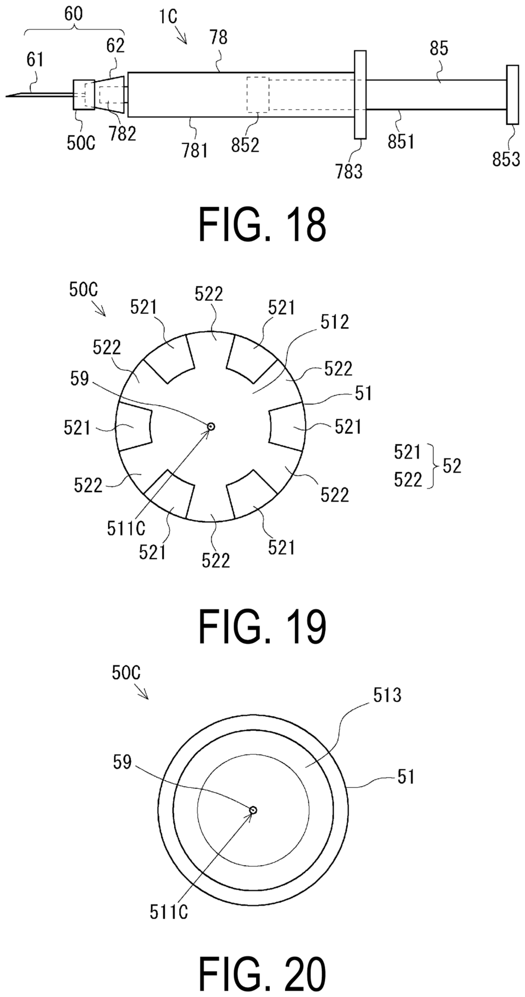

FIG. 18 is a schematic configuration diagram of the injector 1C according to the fourth embodiment, FIG. 19 is a diagram illustrating the distal end side of the positional misalignment prevention tool 50C according to the fourth embodiment, and FIG. 20 is a diagram illustrating the base end side of the positional misalignment prevention tool 50C according to the fourth embodiment. As illustrated in FIG. 18, the injector 1C includes an injector cylinder 78, an injection rod 85, and an injector needle 60.

The injector cylinder 78 includes a substantially cylindrical outer cylinder body 781, and a cylinder distal end portion 782 on the distal end side of the outer cylinder body 781. The cylinder distal end portion 782 is formed in a substantially cylindrical shape having a diameter smaller than that of the outer cylinder body 781. The outer cylinder body 781 and the cylinder distal end portion 782 are connected to each other and thus the internal spaces thereof communicate with each other. That is, the distal end side of the internal space of the outer cylinder body 781 is closed except for a portion communicating with the internal space of the cylinder distal end portion 782. A flange (finger hook portion) 783 that protrudes in the radial direction from the outer peripheral surface of the cylinder is provided at the rear end of the outer cylinder body 781.

The injector cylinder 78 is formed using, for example, a transparent plastic. The material of a first outer cylinder 11 is not particularly limited, and may be, for example, a metal, a glass, or a ceramic, instead of a plastic.

The injection rod 85 includes a rod-shaped rod portion 851, a gasket 852 provided at the distal end of the rod portion 851, and a flange 853 provided at the rear end of the rod portion 851. The distal end side of the injection rod 85 is inserted into the internal space from the rear end of the outer cylinder body 781. The gasket 852 of the injection rod 85 inserted into the internal space of the outer cylinder body 781 is in contact with the inner peripheral surface of the outer cylinder body 781, and in this state, can move (advance and retract) toward the distal end side or the base end side along the inner peripheral surface. That is, the injection rod 85 is held being movable forward and backward in a state where the gasket 852 at the distal end is in contact with the inner peripheral surface of the outer cylinder body 781 and the airtightness of the internal space is maintained.

The injection needle 60 includes a needle tube (ejection portion) 61 and a needle base 62, and the needle tube 61 and the needle base 62 are connected to each other and thus internal spaces thereof communicate with each other. A recessed portion into which the cylinder distal end portion 782 of the injector cylinder 78 is fitted is provided on the base end side of the needle base 62. When the cylinder distal end portion 782 of the injector cylinder 78 is fitted into the recessed portion of the needle base 62 and the injection needle 60 is attached to the injector cylinder 78, the internal space of the injection needle 60 communicates with the internal space of the injector cylinder 78.

For the injector 1C, which is obtained by assembling the injector cylinder 78, the injection rod 85, and the injector needle 60, when the injection rod 85 is moved toward the base end side with respect to the injector cylinder 78 in a state where the distal end of the injector needle 60 is immersed in the ejection solution corresponding to the substance to be injected, the ejection solution is sucked and housed in the injector cylinder 78. Further, for the injector 1C, in a state where the ejection solution is housed in the injector cylinder 78, when the distal end of the injector needle 60 punctures the target region and the injection rod 85 is moved toward the distal end side with respect to the injector cylinder 78, the ejection solution is pushed out and injected into the target region.

The positional misalignment prevention tool 50C of the present embodiment includes the base 51 disposed around the axis 59 along the ejection direction of the ejection solution in a manner that the base 51 surrounds the periphery of the injection needle 60, and the irregular portion 52 provided on the target region side of the base 51.

The base 51 is an annular member and is externally fitted to the distal end side of the needle base 62 with the needle tube 61 of the injector needle 60 passing through an inner space 511C. Note that the positional misalignment prevention tool 50C of the present embodiment is detachably attached by fitting the base 51 and the needle base 62 together, but is not limited thereto, and may be fixed to the needle base 62 by adhesion, welding, or the like. Alternatively, the positional misalignment prevention tool 50C may be formed integrally with the needle base 62.

The surface (base end surface) 513 on the base end side of the base 51 is formed in a tapered shape, following the outer surface (tapered surface) on the distal end side of the needle base 62.

In the irregular portion 52, the plurality of protruding portions 521 are erected on the surface (base surface) 512, of the base 51, facing the target region. Note that the configuration and function of the irregular portion 52 are similar to those of the above-described embodiments.

For the injector 1C of the present embodiment, the distal end side of the injector needle 60 is punctured into the target region during use of the injector 1C, the injector needle 60 is inserted until the positional misalignment prevention tool 50C comes into contact with the symmetrical region, and the injection rod 85 is moved toward the distal end side with respect to the injector cylinder 78 in this state, whereby the ejection solution is injected into the target region. As described above, in the present embodiment, when the ejection solution is injected into the target region, the irregular portion 52 of the positional misalignment prevention tool 50C comes into contact with the surface of the symmetrical region (for example, the surface of the eyeball), and thus it is possible to suppress the misalignment of the injector 1C with respect to the target region.

### Fifth Embodiment

The present embodiment is different from the fourth embodiment in that an injection needle 60D includes a sleeve 63 and a shield 64, and a positional misalignment prevention tool 50D is attached to the sleeve 63. Note that since other portions are the same as those of the fourth embodiment described above, the same elements are denoted by the same reference signs and description thereof will not be repeated.

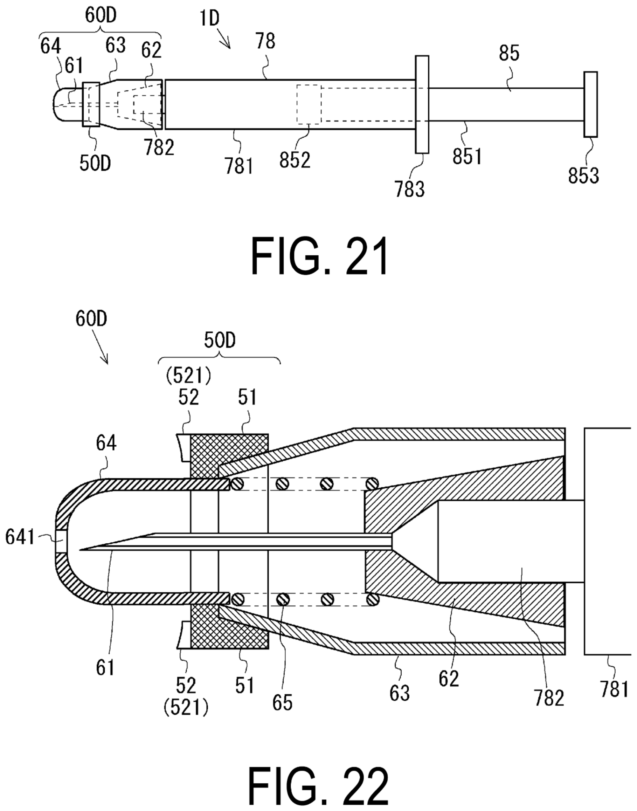

FIG. 21 is a schematic configuration diagram of the injector 1D according to a fifth embodiment, and FIG. 22 is a diagram illustrating a cross section of the distal end side of the injector 1D. In the injector 1D of the present embodiment, the injection needle 60D includes the sleeve 63, the shield 64, and a spring 65 in addition to the needle tube 61 and the needle base 62.

The sleeve 63 is a substantially cylindrical member and houses the needle base 62 and the base end side of the needle tube 61. The sleeve 63 houses at least the base end side of the shield 64 and holds the shield 64 in a manner that the shield 64 is movable forward and backward along the ejection direction of the injector 1D. The shield 64 is a substantially cylindrical member and disposed surrounding the needle tube 61 in the radial direction. A hole 641 through which the needle tube 61 passes is provided on the distal end side of the shield 64. The spring 65 is provided on the base end side of the shield 64, and the shield 64 is biased toward the distal end side. Therefore, when the shield 64 does not receive an external force (when not in use), the shield 64 covers a distal end portion of the needle tube 61. Then, when the shield 64 is pushed toward the base end side during use of the injector 1D, the shield 64 compresses the spring 65 and moves toward the base end side, thereby exposing the distal end portion of the needle tube 61.

The sleeve 63 is connected to the needle base 62, and the injection needle 60D including the sleeve 63 is attached to the injector cylinder 78 by externally fitting the needle base 62 to the cylinder distal end portion 782 of the injector cylinder 78. At this time, the injector needle 60D may be attached in a state where the injector cylinder 78 is filled with the ejection solution, or the injector cylinder 78 may be filled with the ejection solution via the injector needle 60D after the injector needle 60D is attached to the injector cylinder 78.

The positional misalignment prevention tool 50D of the present embodiment includes the base 51 disposed around the axis 59 along the ejection direction of the ejection solution in a manner that the base 51 surrounds the needle tube 61 and the shield 64, and the irregular portion 52 provided on the target region side of the base 51.

The base 51 is an annular member, and is fitted to the distal end side of the sleeve 63 with the needle tube 61 and the shield 64 of the injection needle 60D passing through an inner space 511D. Note that the positional misalignment prevention tool 50D of the present embodiment is detachably attached by fitting the base 51 and the sleeve 63 together, but is not limited thereto, and may be fixed to the sleeve 63 by adhesion, welding, or the like. Alternatively, the positional misalignment prevention tool 50D may be formed integrally with the sleeve 63.

In the irregular portion 52, the plurality of protruding portions 521 are erected on the surface (base surface) 512, of the base 51, facing the target region. Note that the configuration and function of the irregular portion 52 are similar to those of the above-described embodiments.

The injector 1D is pressed with its distal end against the target region during use. At this time, the shield 64 is positioned at the distal end and covers the needle tube 61 until the injector 1D touches the target region. When the shield 64 reaches the target region, the shield 64 abuts against the surface of the target region and stops moving forward, but a portion of the injector 1D other than the shield 64 moves forward relative to the shield 64 while compressing the spring 65. Then, the needle tube 61 is punctured into the target region, the injector 1D is inserted until the position misalignment prevention tool 50D comes into contact with the symmetrical region, and the injection rod 85 is moved toward the distal end side with respect to the injector cylinder 78 in this state, whereby the ejection solution is injected into the target region. As described above, in the present embodiment, when the ejection solution is injected into the target region, the irregular portion 52 of the positional misalignment prevention tool 50D comes into contact with the surface of the symmetrical region (for example, the surface of the eyeball), and thus it is possible to suppress the misalignment of the injector 1C with respect to the target region.

Although the embodiments of the positional misalignment prevention tool 50 according to the present disclosure have been described above, each of the aspects disclosed in the present specification can be combined with any other features disclosed therein.

### Reference Signs List

1 Injector
2 Housing
3 Power cable
4 Connector
7 Socket
10 Injector assembly
20 Actuator
20a Combustion chamber
21 Body
22 Igniter
26 Male thread portion
27 Opening
30 Attachment
31 Body
32 Female thread portion
35 Third region
36 Female thread portion
40 Piston
50 Positional misalignment prevention tool
50A Positional misalignment prevention tool
51 Base
52 Irregular portion
59 Axis
70 Container
70A Container
70c Nozzle portion
70c1 Tapered portion
70c2 Distal end portion
70c3 Distal end side outer surface
71 Container holder
73 Distal end surface
74 Male thread portion
75 Housing space
75a Inner wall surface
76 Flow path
77 Ejection port
80 Plunger
81 Plunger rod
82 Stopper portion
91 Predetermined curved surface
511 Inner space
512 Base surface
521 Protruding portion
522 Recessed portion
523 Contact surface

## Claims

1. A positional misalignment prevention tool configured to suppress misalignment of an injector with respect to a target region of an eyeball being an injection target, the injector being configured to eject a substance to be injected from an ejection portion to the target region, the positional misalignment prevention tool comprising:
a base disposed around an axis along an ejection direction of the substance to be injected, the base surrounding a periphery of the ejection portion; and
an irregular portion provided on the target region side of the base.

2. The positional misalignment prevention tool according to claim 1, wherein
the injector is a needleless injector configured to inject the substance to be injected into the target region without using an injection needle, by ejecting the substance to be injected from an ejection port being the ejection portion.

3. The positional misalignment prevention tool according to claim 2, wherein
the needleless injector includes
a housing portion provided with a housing space in which the substance to be injected is housed and the ejection port from which the substance to be injected is ejected to the target region, and
the base is attached to the housing portion of the needleless injector.

4. The positional misalignment prevention tool according to claim 2, wherein
the needleless injector includes
a housing portion provided with a housing space in which the substance to be injected is housed and the ejection port from which the substance to be injected is ejected to the target region, and
a holding portion configured to hold the housing portion, and
the base is attached to the holding portion of the needleless injector.

5. The positional misalignment prevention tool according to claim 1, wherein
the ejection portion of the injector includes an injection needle, and
the base is disposed at a position surrounding the injection needle around the axis.

6. The positional misalignment prevention tool according to any one of claims 2 to 5, wherein
the irregular portion includes a plurality of protrusions around the axis, and
a contact surface of each of the protrusions that is to come into contact with the eyeball is formed along a predetermined curved surface corresponding to the eyeball.

7. An injector configured to eject a substance to be injected from an ejection portion to a target region of an eyeball being an injection target, the injector comprising:
a housing portion provided with a housing space in which the substance to be injected is housed and the ejection portion from which the substance to be injected is ejected to the target region; and
a positional misalignment prevention tool configured to suppress misalignment of the injector with respect to the target region, wherein
the positional misalignment prevention tool includes
a base disposed around an axis along an ejection direction of the substance to be injected, the base surrounding a periphery of the ejection portion, and
an irregular portion provided on a surface, of the base, facing the target region.

8. The injector according to claim 7, wherein
the injector is a needleless injector configured to inject the substance to be injected into the target region without using an injection needle by ejecting the substance to be injected from an ejection port being the ejection portion.

9. The injector according to claim 8, wherein
the base is attached to the housing portion of the needleless injector.

10. The injector according to claim 8, further comprising:
a holding portion configured to hold the housing portion, wherein
the base is attached to the holding portion of the needleless injector.

11. The injector according to claim 7, wherein
the ejection portion includes an injection needle, and
the base is disposed at a position surrounding the injection needle around the axis.

12. The injector according to any one of claims 7 to 11, wherein
the irregular portion includes a plurality of protrusions around the axis, and
a contact surface of each of the protrusions that is to come into contact with the eyeball is formed along a predetermined curved surface corresponding to the eyeball.
